# EUROPEAN PATENT SPECIFICATION

(11) **EP 2 219 698 B1**
(45) Date of publication and mention of the grant of the patent: **22.08.2018**
(21) Application number: 08865704.4
(22) Date of filing: 19.12.2008
(51) Int. Cl.: A61L 29/06

(54) **CROSS-LINKED POLYMERS IN MEDICAL DEVICES**
VERNETZTE POLYMERE IN MEDIZINISCHEN VORRICHTUNGEN
POLYMÈRES RÉTICULÉS DANS DES DISPOSITIFS MÉDICAUX

(30) Priority: 21.12.2007 EP 07024954
(43) Date of publication of application: 25.08.2010
(73) Proprietor: Abbott Laboratories Vascular Enterprises Limited, Dublin 2 (IE)
(72) Inventor: GÜNTER, Lorenz, 72072 Tübingen (DE)
(74) Representative: Peters, Hajo
(86) International application number: PCT/EP2008/010949
(87) International publication number: WO 2009/080323

(56) References cited:
- WO-A-98/55171
- WO-A-2007/054364
- US-A1- 2003 195 490
- US-A1- 2007 149 690
- US-B1- 6 875 197
- EICHHORN K-J- ET AL: "CHARACTERIZATION OF LOW MOLECULAR WEIGHT CARBOXYL-TERMINATED POLYAMIDES OBTAINED BY REACTIVE EXTRUSION OF POLYAMIDE 6 WITH TRIMELLITIC ANHYDRIDE" 19 December 1996 (1996-12-19), JOURNAL OF APPLIED POLYMER SCIENCE, JOHN WILEY AND SONS INC. NEW YORK, US, PAGE(S) 2053 - 2060 , XP000637743 ISSN: 0021-8995 cited in the application abstract

## Description

### Field of the invention

The present invention refers to medical devices comprising cross-linked modified polyamides especially to stent carrying balloon catheters, for use in angioplasty and other procedures of vessel repair.

### Background of the invention

Angioplasty is an efficient and successful method of opening stenosis in the vascular system. In a popular form of angioplasty, a balloon catheter is advanced through the vascular system until the balloon, which is carried at the distal end of a catheter shaft, and which may carry an expandable stent, is positioned across the stenosis or damaged vessel. By inflating the balloon pressure is applied to the obstruction which is moved by pressing it against the inner wall of the vessel, whereby the vessel is opened for improved flow. Due to the expansion of the balloon, the stent, which - if used - is situated on the balloon, is also expanded for aiding in repairing the vessel wall and hindering obstruction. As a last step the stent is then released by deflating the balloon reducing its circumference until refolding of the balloon occurs followed by removal of the balloon and catheter from the vessel.

There are various types of balloon catheters. One type is fed over a guide wire (i.e., "over-the-wire" catheters) and another type serves as its own guide wire ("fixed -wire" catheters). There have been developments of variations of these two basic types: the so-called "rapid exchange" type, "innerless" catheters, and others. The term "balloon catheter" as defined in this invention is meant to include all the various types of angioplasty catheters which carry a balloon for performing angioplasty and any other type of stent carrying balloon catheter. Balloon catheters also have a wide variety of inner structure, such as different lumen design, of which there are at least three basic types: triple lumen, dual lumen and co-axial lumen. All these varieties of internal structure and design variations are included in the definition "balloon catheter" herein.

If a balloon catheter is used in percutaneous transluminal angioplasty (PTA) or percutaneous transluminal coronary angioplasty (PTCA), it is typically advanced through a guide catheter to a preselected vessel location, such as the aorta, for example. Using fluoroscopy, the surgeon advances the catheter until the balloon is located across the stenosis or obstruction. This may involve the use of a guide wire over which the catheter is moved or alternatively the catheter may act as its own guide wire.

Polyamides or polyamide elastomers have been used in the polymer industry for a long time and - due to their enormous range of possible applications - are found in many branches of industrial products. Recently in the area of medicinal devices good use has been made of these materials especially in devices/implants like the balloons on a balloon catheter. The most popular polyamides used include different sorts of Nylons or Copolymers such as PEBA. Even though these materials have certainly been used successfully, due to the strains put on the materials and the necessity to improve their characteristics in the light of growing experience coming from increasing numbers of treated patients, there clearly is a need for improved materials allowing for an effective treatment of the patient minimizing risks, preferably with an economical production process.
- WO2007/054364 A (by the Applicant) discloses a catheter comprising an outer tube, an inner tube and a balloon with the inner tube comprising an elastic segment.
- US 2003/0195490 A1 (by Cook Inc., US) is drawn to a medical device with part of it formed of a mixture of a polamide and cross-linking reagent.
- US 6,875,197 B1 (by Advanced Cardiovascular Systems Inc. US) describes an inflatable member formed by adding a multifunctional agent to a soft polymer.
- WO98/55171 (by L. Acquarulo, US) is drawn to a cross-linked block copolymer.
- US 2007/0149690 A1 (by Bostson scientific Scimed, Inc. US) is describing a functionalized block copolymer.
- EICHHORN K-J- ET AL: (1996-12-19), J. APPL. POLYMER SCI., p. 2053 - 2060 deals with polyamides obtained by reactive extrusion of a polyamide with trimellitic anhydride.

With the focus of this invention on balloon material for balloon catheters one of the main parameters of a balloon is compliance, the change of the balloon diameter with rising inflation pressure; as used herein three categories are being identified:
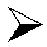 Non-compliant (NC) with a diameter increase of up to 0.55% per bar;
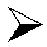 Semi-compliant (SC) with a diameter increase of between 0.55 to 0.8 % per bar;
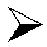 Compliant with a diameter increase over 0.8 % per bar
as the balloon is pressurized from an inflation pressure between the nominal pressure and rated burst pressure.

While a certain level of compliance is needed to allow the compression of the arterio-sclerotic plaque in a vessel, an amount of pressure expressed on the stenosis as executed by a more non-compliant balloon is also needed. As also semi-compliant and compliant balloons are more prone to failure during PTA or PTCA and also "dog-boning", an inflation of the balloon outside the stenotic area of the vessel resulting sometimes in devastating stress on the healthy part of the vessel, mostly a more non-compliant parameter is wanted.

Additional attributes wanted in medical balloons are dimensional stability in balloon material as this allows for a safe use e.g. in PTA or PTCA as the balloon keeps his form and also - for certain uses a low difference in dimension and compliance between a first and a second inflation of the balloon, as well as a reduced creeping behavior during balloon inflation.

### Summary of the invention

It is an object of the current invention to provide new medical devices using polymers showing the attributes wanted in their specific area of use. As the special focus of this invention is on the search for new materials to be used in medical balloons for balloon catheters used in PTA (percutaneous transluminal angioplasty) or PTCA (Percutaneous transluminal coronary angioplasty) a material with suitable compliance (most preferably being easily adapted by choosing reaction conditions when producing the polymeric material) needs to be identified, which preferably also show other wanted attributes like increased dimensional stability, a low difference in dimension and compliance between a first and a second inflation of the balloon, as well as reduced creeping behavior during balloon inflation.

The invention thus refers to a medical device comprising a cross-linked modified polyamide, especially to a balloon attached to a balloon catheter like those used in PTA/PTCA/angioplastic applications. Medical devices, especially medical balloons constructed from this cross-linked modified polyamides, especially if cross-linked after forming the balloon do show reduced creeping behavior during balloon inflation, and therefore also show an increased dimensional stability. Furthermore these materials show a linear compliance a low difference between a first and a second inflation of the balloon, with advantageously also the slope of the compliance curve is being easily adjustable by changing the cross-linking density (either by choosing the appropriate amount of cross-linking compounds or by adapting the energy used for curing/cross-linking).

### Detailed Description of the invention

The use of stents, balloons, catheters and other medical devices etc. in minimal invasive surgery, especially in the cardiovascular field, has in the last years shown a high growth. As a consequence the need for useful materials fulfilling highly specialized needs in the field of different medicinal devices has clearly risen in a technical area, which traditionally is more governed by bulk products. Especially in the field of vascular balloons there was a clear desire for an elastomer, which is on one hand flexible enough to be introduced into a vascular environment without causing damage, while on the other hand being stable and rigid enough, especially in the moment of actual surgery, inflation in the vessel, to not be extended too much inside the vessel. Especially a suitable compliance, the change of the balloon diameter with rising inflation pressure, especially with a flat rise in the compliance curve (pressure/diameter) is needed.

There are 3 kinds of material used nowadays for medical devices, especially balloons.
a) Nylon: Nylon, coming in different sorts, especially Nylon-12, are not very flexible and/or have a relatively high water absorption. Especially the lack of flexibility is often considered as a drawback in medical devices using Nylon.
b) PEBA: PEBA (e.g. PEBAX®) are often too flexible and/or have a relatively high water absorption In addition PEBA lacks stability, especially in regards to the thermo-oxidation and/or sometimes also lack dimensional stability.
c) Blend of a) and b): The need for a compromise between the higher rigidity of Nylon and higher flexibility of PEBA has already resulted in blends being used. A disadvantage of blends is that the phases tend to show phase separation that leads to unstable morphology.

None of these materials does show an optimal behavior, especially if used in medical balloons, e.g. they show differences between first and second inflation, so that still there is a high need for appropriate material for medical devices, especially for medical balloons.

The invention thus refers to a medical device comprising a cross-linked modified polyamide. The medical device according to the invention is a balloon. Most desirably the medical device according to the invention is a balloon attached to a balloon catheter, like a catheter for angioplastic applications like PTA or PTCA.

These medical devices, especially medical balloons constructed from these cross-linked modified polyamides - especially if cross-linked after forming the balloon - do show reduced creeping behavior during balloon inflation, and therefore also show an increased dimensional stability. Furthermore these materials show a linear compliance a low difference between a first and a second inflation of the balloon, with advantageously also the slope of the compliance curve is being easily adjustable by changing the cross-linking density (either by choosing the appropriate amount of cross-linking compounds or by adapting the energy used for curing/cross-linking).

"Modified polyamides" is defined herein as polyamides, e.g. like Nylon, like Nylon 12, having been modified by reaction with a crosslink-reactive compound.

A "crosslink-reactive compound " is a compound containing at least one reactive site, allowing cross-linking or reaction with cross-linkers, like e.g. mono- or bifunctional crosslink reactive compounds or e.g. TAIC. Usually bifunctional substances will be introduced either at the end of the polymer chain or in its backbone as described by Hartkopf et al. (Angew. Makromol. Chem. 169 (1989) 193-209).

In the context of this invention a "reactive site allowing cross-linking or reaction with cross-linkers" is defined as a reactive site in a molecule that either by itself or by reaction with a cross-linker (a cross-linking agent) allows the cross-linking between two polyamide chains (see below). A highly popular example of such a reactive site is a C=C double bond being introduced by the modification (see above) into a polyamide chain. Another example is/are the reactive double bonds in TAIC.

In the context of this invention "functional" is defined as showing functional groups. Accordingly a "mono- or bifunctional compound" has one or two "functional groups". "Functional group" is defined as a chemical substituent bound to a block/segment of a polymer allowing the coupling (covalent binding) of this block/segment of polymer to another block (segment) of polymer or to a coupling reagent/linker. Examples of functional groups include epoxides, OH, COOH, NH₂, or others.

"Cross-linked polyamides" is defined as polyamides, e.g. like Nylon (e.g. Nylon 12), in which the linear chains out of which the polyamides are build, are cross-connected to each other. Thus, at least one polyamide chain is cross-connected (chemically bound) to at least one other polyamide chain in at least one position, allowing also for more bounds between the same polyamide chain and between more than 2 chains.

According to the two definitions given above "cross-linked modified polyamides" is defined as "modified polyamides", e.g. like modified Nylon (e.g. modified Nylon 12), in which the linear chains out of which the polyamides are build, are cross-connected to each other. Thus - after modification of the original polyamide - at least one modified polyamide chain is cross-connected (chemically bound) - through the sites introduced by modification - to at least one other polyamide chain in at least one position, allowing also for more bonds between the same polyamide chains and between more than 2 chains.

"(Medical) balloon or balloon material" in the context of this invention especially means a balloon like those used in coronary balloon angioplasty and the material used for these balloons, especially balloon catheters. In this, e.g. a balloon catheter is inserted into an artery or other lumen and advanced to e.g. a narrowing in a coronary artery. The balloon is then inflated to enlarge the lumen.

"Stent" means an elongate implant with a hollow interior and at least two orifices and usually a circular or elliptical, but also any other, cross section, preferably with a perforated, lattice-like structure that is implanted into vessels, in particular blood vessels, to restore and maintain the vessels patent and functional.

"Graft" means an elongate implant with a hollow interior and with at least two orifices and usually circular or elliptical, but also any other, a cross section and with at least one closed polymer surface which is homogeneous or, optionally, woven from various strands. The surface preferably is impermeable to corpuscular constituents of blood and/or for water, so that the implant serves as a vascular prosthesis and is usually employed for damaged vessels or in place of vessels.

"Stent graft" means a connection between a stent and a graft. A stent graft preferably comprises a vascular prosthesis reinforced with a stent (both as defined above), wherein a polymer layer is homogeneous or, optionally, woven, knitted plaited etc. from various strands and is either impermeable for corpuscular constituents of blood and/or for water or can also be permeable. More preferably, the stent has on at least 20% of its surface a perforated (lattice-like), preferably metallic, outer layer and at least one closed polymer layer that is located inside or outside the stent outer layer. The closed polymer layer may be homogeneous or, optionally, woven from various strands, and is impermeable for corpuscular constituents of blood and/or for water. Optionally, where the closed polymer layer is disposed inside the metallic outer layer, a further perforated (lattice-like), preferably metallic, inner layer may be located inside the polymer layer.

"Graft connector" means an implant that connects at least two hollow organs, vessels or grafts, consists of the materials defined for grafts or stent grafts and/or has the structure defined for the latter. Preferably, a graft connector has at least two, three or four, orifices, arranged, for example, as an asymmetric "T" shape.

"Catheter" means a tubular instrument intended for introduction into hollow organs. More preferably, a catheter may be designed for use in guiding other catheters, or for angiography, ultrasound imaging, or - especially - balloon catheters for dilatation or stent delivery. This includes also a "Catheter pump" meaning a catheter provided on its tip with a propeller able to assist the pumping of the myocardium.

The cross-linked modified polyamide is obtainable by a process in which at least one step is executed using reactive extrusion. Reactive extrusion is described in DD 276 290 A1 and Eichhorn et al. (Journal of Applied Polymer Science, Vol. 62, 2053-2060 (1996) mentioned above.

For the medical device according to the invention being a balloon comprising a cross-linked modified polamide the cross-linked modified polyamide is obtainable by a process in which the polyamide is
- in a first step modified with either (a) a bifunctional or monfunctional crosslink-reactive compound comprising at least one reactive site allowing cross-linking or reaction with cross-linkers or (b) with triallyl isocyanurate (TAIC) or derivatives thereof, the first step is executed using reactive extrusion, and
- in a second step is undergoing a cross-linking reaction by treatment with at least one form of energy, with the second step being executed after the balloon was formed in a blow molding process,
with the polyamide being Nylon and the bifunctional or monofunctional crosslink-reactive compound being a linear compound comprising a C=C-doublebond.

In a preferred embodiment of the medical device according to the invention the (medical) balloon is consisting of the cross-linked modified polyamide.

Preferably the energy being used for the second step, cross-linking reaction, is irradiation and/or thermal energy, preferably is heating, UV-irradiation, electron beam (e-beam) or gamma irradiation.

In a further preferred embodiment of the medical device according to the invention the cross-linking reaction is executed as vulcanisation reaction using sulphur as crosslinker and/or radical reaction using UV curing agents optionally in combination with acrylates or photomer polymers. Desirably the UV curing agents are selected from benzophenone, or hydroxycyclohexy phenyl ketone, or stilbene derivatives, and/or the acrylates are selected from trimethylpropane triacylates. Radical reactions with UV-curing agents like benzophenone, hydroxycyclohexyl-phenyl-ketone or stilbene derivatives are described by Lobmaier et al. (J.Mol.Recog. 8 (1995) 146-150).

In a non-claimed embodiment of the medical device the modified polyamide is described by any of formulas la, lb or Ic 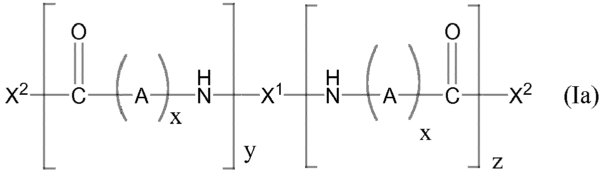 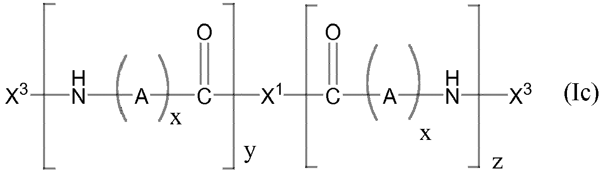 with
X¹ being a bifunctional crosslink-reactive compound;
X² being a bifunctional crosslink-reactive compound,
   a monfunctional crosslink-reactive compound, or O-B or O-B'; and
X³ being a bifunctional crosslink-reactive compound,
   a monfunctional crosslink-reactive compound, or H;
or 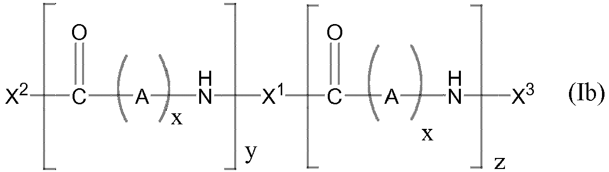 either with
X¹ being a bifunctional crosslink-reactive compound;
   while
X² is a bifunctional crosslink-reactive compound,
   a monfunctional crosslink-reactive compound, or O-B or O-B'; and
X³ is a bifunctional crosslink-reactive compound,
   a monfunctional crosslink-reactive compound, or H;
or with
X¹ being a chemical bond;
   while one of
X² or X³ is a bifunctional crosslink-reactive compound, or a monfunctional crosslink-reactive compound;
   while for the other of X² or X³ applies that
X² is a bifunctional crosslink-reactive compound,
   a monfunctional crosslink-reactive compound, or O-B or O-B'; or
X³ is a bifunctional crosslink-reactive compound,
   a monfunctional crosslink-reactive compound, or H;
wherein for la, Ic or lb
A is a divalent, branched or linear, saturated or non-saturated, optionally substituted hydrocarbon chain, with optionally one carbon atom being replaced by NH, O or S;
B and B' independently from one another are selected from H or C₁₋₄-Alkyl;
x is a natural number between 1 and 24;
y is a natural number ≥1.
z is a natural number ≥1 or 0.

The examples in the following section are illustrative.

### Examples:

### Example 1: Cross-linking with TAIC (Triallyl isocyanurate)

In a first step 50 g dried Nylon 12 (with a molecular weight of approx. 26000 g/mol) is mixed with 2.5 g of TAIC (Triallyl isocyanurate) (MW 259.27) in an extruder and extruded by way of an reactive extrusion as described in DD 276 290 A1 and Eichhorn et al. (Journal of Applied Polymer Science, Vol. 62, 2053-2060 (1996).

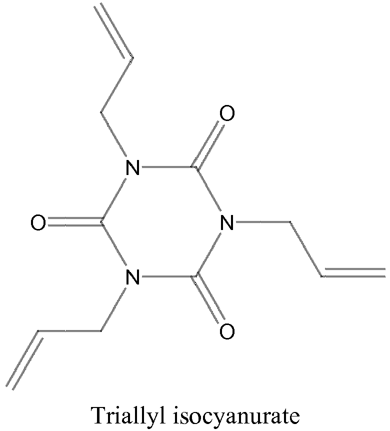

This modified Polyamide material is extruded and from the extruded polymeric material a balloon is formed in a blow molding process. Following that the balloon is cured (the modified polyamides cross-linked) by electron beam (E-beam).

This process of modifying and cross-linking of Nylon by TAIC or its derivatives is described in detail in Brocka and Ehrenstein (Kunststoffe 11 (2004) 88-94) and Duk Won Lee, Däniken (Kunststoffe 6 (2001) 78-80).

### Example 2: Cross-linking with but-2-enedioic acid under vulcanisation

In a first step 50 g dried Nylon 12 (with a molecular weight of approx. 26000 g/mol) is mixed with 2.5 g of but-2-enedioic acid in an extruder, with sulphur being added, and extruded by way of reactive extrusion (see above).

This modified Polyamide material is extruded and from the extruded polymeric material a balloon is formed in a blow molding process. Following that the balloon is cured (the modified polyamides cross-linked by vulcanization) by heating, following the reaction described below in a general form:

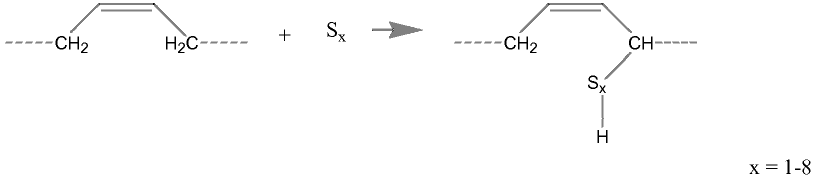

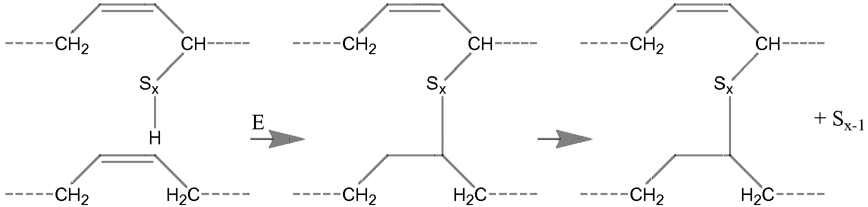

### Example 3: Cross-linking with 1,4-butylene diamine using UV curing reagents

In a first step 50 g dried Nylon 12 (with a molecular weight of approx. 26000 g/mol) is mixed with 1.25 g of 1,4-butylene diamine in an extruder, with benzophenone (with or without trimethylpropane triacrylate) being added, and extruded by way of reactive extrusion.

This modified Polyamide material is extruded and from the extruded polymeric material a balloon is formed in a blow molding process. Following that the balloon is cured (the modified polyamides cross-linked by vulcanization) by UV-radiation.

Radical reactions with UV-curing agents like benzophenone, hydroxycyclohexyl-phenyl-ketone or stilbene derivatives are described by Lobmaier et al. (J.Mol.Recog. 8 (1995) 146-150).

### Example 4: Cross-linking with 4-Amino-but-2-enoic acid using UV curing reagents

In a first step 50 g dried Nylon 12 (with a molecular weight of approx. 26000 g/mol) is mixed with 5 g of 4-Amino-but-2-enoic acid in an extruder, with hydroxycyclohexyl-phenyl-ketone (with or without trimethylpropane triacrylate) being added, and extruded by way of reactive extrusion.

This modified Polyamide material is extruded and from the extruded polymeric material a balloon is formed in a blow molding process. Following that the balloon is cured (the modified polyamides cross-linked by vulcanization) by UV-radiation.

## Claims

1. Medical device being a balloon comprising a cross-linked modified polyamide,
wherein the cross-linked modified polyamide is obtainable by a process in which the polyamide is
• in a first step modified with either (a) a bifunctional or monofunctional crosslink-reactive compound comprising at least one reactive group allowing reaction with crosslinkers or (b) with triallyl isocyanurate (TAIC) or derivatives thereof, the first step is executed using reactive extrusion, and
• in a second step is undergoing a cross-linking reaction by treatment with at least one form of energy,
wherein the second step is executed after the balloon was formed in a blow molding process; and
wherein the polyamide is Nylon and the bifunctional or monofunctional crosslink-reactive compound is a linear compound comprising a C=C-doublebond.

2. Medical device according to claim 1, wherein the medical device is a balloon attached to a balloon catheter.

3. Medical device according to claim 2, wherein the balloon is consisting of the cross-linked modified polyamide.

4. Medical device according to claim 1, wherein the energy being used for the cross-linking reaction is irradiation and/or thermal energy, preferably is heating, UV irradiation, electronic beam (e-beam) or gamma irradiation.

5. Medical device according to claim 1, wherein the cross-linking reaction is executed as vulcanisation reaction using sulphur as crosslinker and/or radical reaction using UV curing agents optionally in combination with acrylates or photomer polymers.

6. Medical device according to claim 5, wherein the UV curing agents are selected from benzophenone, or hydroxycyclohexy phenyl ketone, or stilbene derivatives, and/or the acrylates are selected from trimethylpropane triacylates.

## Patentansprüche

1. Medizinische Vorrichtung, die ein Ballon ist, der ein vernetztes modifiziertes Polyamid umfasst,
wobei das vernetzte modifizierte Polyamid durch ein Verfahren erhältlich ist, in dem das Polyamid
• in einem ersten Schritt entweder mit (a) einer bifunktionellen oder monofunktionellen vernetzungsreaktiven Verbindung, die mindestens eine reaktive Gruppe umfasst, welche eine Reaktion mit Vernetzern erlaubt, oder (b) mit Triallylisocyanurat (TAIC) oder Derivaten davon modifiziert wird, wobei der erste Schritt unter Anwendung reaktiver Extrusion durchgeführt wird, und
• in einem zweiten Schritt einer Vernetzungsreaktion durch Behandlung mit mindestens einer Form von Energie unterzogen wird,
wobei der zweite Schritt durchgeführt wird, nachdem der Ballon in einem Blasformverfahren gebildet wurde; und
wobei das Polyamid Nylon ist, und die bifunktionelle oder monofunktionelle vernetzungsreaktive Verbindung eine eine C=C-Doppelbindung umfassende lineare Verbindung ist.

2. Medizinische Vorrichtung nach Anspruch 1, wobei die medizinische Vorrichtung ein an einem Ballonkatheter befestigter Ballon ist.

3. Medizinische Vorrichtung nach Anspruch 2, wobei der Ballon aus dem vernetzten modifizierten Polyamid besteht.

4. Medizinische Vorrichtung nach Anspruch 1, wobei die für die Vernetzungsreaktion verwendete Energie Bestrahlung und/oder Wärmeenergie ist, vorzugsweise Erwärmung, UV-Bestrahlung, Elektronenstrahl (E-Strahl) oder Gammabestrahlung ist.

5. Medizinische Vorrichtung nach Anspruch 1, wobei die Vernetzungsreaktion als Vulkanisationsreaktion unter Anwendung von Schwefel als Vernetzer und/oder Radikalreaktion unter Anwendung von UV-Härtungsmitteln gegebenenfalls in Kombination mit Acrylaten oder Photomerpolymeren durchgeführt wird.

6. Medizinische Vorrichtung nach Anspruch 5, wobei die UV-Härtungsmittel aus Benzophenon oder Hydroxycyclohexylphenylketon oder Stilbenderivaten ausgewählt sind, und/oder die Acrylate aus Trimethylpropantriacylaten ausgewählt sind.

## Revendications

1. Dispositif médical étant un ballonnet comprenant un polyamide modifié réticulé,
dans lequel le polyamide modifié réticulé peut être obtenu par un procédé dans lequel le polyamide
• dans une première étape est modifié avec soit (a) un composé réactif réticulable bifonctionnel ou monofonctionnel comprenant au moins un groupe réactif permettant la réaction avec des agents de réticulation soit (b) avec de l'isocyanurate de triallyle (TAIC) ou des dérivés de celui-ci, la première étape étant exécutée à l'aide d'une extrusion réactive, et
• subit dans une deuxième étape une réaction de réticulation par traitement avec au moins une forme d'énergie,
dans lequel la deuxième étape est exécutée après la formation du ballonnet dans un procédé de moulage par soufflage ; et
dans lequel le polyamide est Nylon, et le composé réactif réticulable bifonctionnel ou monofonctionnel est un composé linéaire comprenant une double-liaison C = C.

2. Dispositif médical selon la revendication 1, dans lequel le dispositif médical est un ballonnet attaché à un cathéter à ballonnet.

3. Dispositif médical selon la revendication 2, dans lequel le ballonnet est constitué du polyamide modifié réticulé.

4. Dispositif médical selon la revendication 1, dans lequel l'énergie utilisée pour la réaction de réticulation est une irradiation et / ou une énergie thermique, de préférence un chauffage, une irradiation UV, un faisceau électronique (faisceau électronique) ou une irradiation gamma.

5. Dispositif médical selon la revendication 1, dans lequel la réaction de réticulation est réalisée comme réaction de vulcanisation utilisant du soufre en tant qu'agent de réticulation et / ou réaction radicalaire en utilisant des agents de durcissement UV, éventuellement en combinaison avec des acrylates ou des polymères photomères.

6. Dispositif médical selon la revendication 5, dans lequel les agents de durcissement aux UV sont choisis parmi la benzophénone, ou l'hydroxycyclohexy-phénylcétone, ou les dérivés de stilbène, et / ou les acrylates sont choisis parmi les triacylates de triméthylpropane.
